# EUROPEAN PATENT SPECIFICATION

(11) **EP 0 458 918 B1**
(45) Date of publication and mention of the grant of the patent: **07.05.1997**
(21) Application number: 90916994.8
(22) Date of filing: 14.11.1990
(51) Int. Cl.: G01N 33/569, C12N 15/34, C07K 7/00, C12P 21/00, G01N 33/543

(54) **METHOD FOR OBTAINING A RECOMBINANT PROTEIN AND ITS UTILIZATION IN THE ASSAY FOR THE AFRICAN SWINE PEST VIRUS (ASPV)**
VERFAHREN ZUR HERSTELLUNG EINES REKOMBINANTEN PROTEINS UND SEINE VERWENDUNG IN DER BESTIMMUNGSMETHODE DES AFRIKANISCHEN SCHWEINEPESTVIRUS
PROCEDE D'OBTENTION D'UNE PROTEINE RECOMBINANTE ET SON UTILISATION POUR LA DETECTION DU VIRUS DE LA PESTE PORCINE AFRICAINE (VPPA)

(30) Priority: 20.12.1989 ES 8904300
(43) Date of publication of application: 04.12.1991
(73) Proprietor: UNIVERSIDAD DE OVIEDO, 33003 Oviedo (ES); CONSEJO SUPERIOR DE INVESTIGACIONES CIENTIFICAS, E-28006 Madrid (ES)
(72) Inventor: LOPEZ OTIN, Carlos Facultad de Medicina, E-33007 Oviedo (ES); PEREZ FREIJE, José, Maria Facultad de Medicina, E-33007 Oviedo (ES); VINUELA DIAZ, Eladio Instit.de Biologia Molecular, Canto Blanco E-28049 Madrid (ES)
(74) Representative: Ungria Lopez, Javier
(86) International application number: ES9000043
(87) International publication number: WO9109313

(56) References cited:
- US-A- 4 735 800
- Virology, volumen 175, April 1990, Academic Press Inc., C. Lopez Otin et al.: "Mapping and sequence of the gene coding for protein p72, the major capsid protein of African swine fever virus", paginas 477-484
- Chemical Abstracts, volumen 106, 1987, (Columbus, Ohio, US), ver pagina 592
- Nature, volumen 313, 28 Febrero 1985, K. Jacobs et al.: "Isolation and characterization of genomic and cDNA clones of human erythropoietin", paginas 806-810

## Description

African Swine Fever is an infectious disease of the domestic pig which has caused considerable economic losses in our country for some thirty years. The non-existence of a vaccine capable of inducing a neutralizing immunologic response makes the availability of a safe and efficient diagnostic method an indispensable condition for effective control of the disease. The invention submitted endeavours to provide a rapid, simple, economic and efficient means for disease detection.

The methods currently used in the diagnosis of African Swine Fever (ASF) are based on immunoenzyme assays utilizing as an antigen extracts from infected cells enriched in the viral p72 protein (C. Vela et al., ES-A-539611, 1986.) Preparations for this type are highly contaminated with cell proteins and components from the culture medium, which gives rise to frequent errors in the diagnosis and particularly to false positives. In addition, this way of obtaining the antigen implies the constant handling of the infectious virus, which is dangerous as well as antieconomical, as it requires costly safety facilities with correspondingly lower profit rates. In our opinion the most efficient alternative choice of an antigen would be to use protein p72 itself, in purified condition and produced with adequate genetic engineering techniques. Taking this idea as a starting point, the authors of the invention, after implementing the respective experimental studies, have achieved the aforementioned objectives as expressed in the different aspects of the invention.

US-A-4 735 800 discloses methods and compositions for the cloning of Rift Valley Fever Virus genes in single cell host organisms as well as methods for culturing said single cell organisms to produce Rift Valley Fever Virus gene profucts useful in the preparation of vaccines against Rift Valley Fever Virus.

K. Jacobs et al.: "Isolation and characterization of genomic and cDNA clones of human erythropoietin" (1985), Nature 313:806-810 discloses a method a method of providing a nucleotide probe for human erythropoietin as well as preparing recombinant protein therewith.

Although said two latter prior art documents disclose methods relating to recombinant DNA technology, they are unsuitable to teach any specific conditions for applying said technology in respect of providing a reliable method to prepare an ASFV test method.

The invention as claimed is intended to provide a process for preparing a low-cost, reliable and safe test method for African Swine Fever Virus (ASFV.)

The process comprises the following stages
(a) purifying protein p72 from a viral capside and obtaining partial amino acid sequence T-P-D-D-P-G-A-M-M-I-T-F
(b) identifying the gene encoding said protein by means of radioactively labelled oligonucleotide probe containing an oligonucleotide mixture, whose sequence has been derived from the partial amino acids sequence obtained under (a) above;
(c) identifying the complete nuecleotide sequence of said gene;
(d) expressing said gene in E.coli BL21(DE3) by means of a pAR3038 plasmid whereby the p72 protein located in EcoRIB fragment of the genome of the African Swine Fever Virus is expressed by cloning thereof in said plasmid, to obtain a recombinant pS72a plasmid, and transforming said plasmid with said bacterial host;
(e) culturing said recombinant plasmid and purifying protein p72 from the extracts obtained;
(f) binding the purified protein onto microtiter plates and incubating it together with pig sera samples to be tested as to the presence of antibodies against African Swine Fever Virus.

The first objective was addressed to the identification of the gene encloding protein p72 of ASFV. To this end, the first stage implied purification of the protein and obtaining partial amino acid sequences.

Protein p72 was purified by means of molecular size exclusion HPLC from the extracellular virus obtained through gradient centrifuging, The virus (approx. 1 mg. of the total protein) was dissolved in 500 µl buffer, containing 0.1 M ammonium acetate, ph 5, 2% B-mercaptoethanol and 1% SDS. It was heated to 100° C for 2 minutes and chromatographed on a filtration HPLC column equilibrated and then eluted with 0.1 M ammonium acetate in the presence of 0.1% SDS. Fractions of 1.0 ml were collected and analyzed by electrophoresis in a polyacrylamide and SDS gel. The fractions containing the purified p72 protein were subsequently digested with trypsin for 18 hours at 37° C and at an enzyme/substrate ratio of 1/50. The resulting peptides were fractionated by reverse-phase HPLC on a column equilibrated with 0.1% trifluoroacetic acid at room temperature and a constant flow rate of 1.5 ml/min. The purified peptides were subjected to Edman's sequential degradation in the presence of polybrene and utilizing a pulsed liquid-gas-phase sequenator. For the identification of the gene coding for protein p72 the probe used was an oligonucletide mixture whose sequence had been derived from the partial amino acid sequence previously obtained. The heptapeptide corresponding to the 5-11 amino acids of the T35 peptide was chosen (T-P-D-D-P-G-A-M-M-I-T-F), which showed the lowest degree of codon degeneracy among the sequenced peptides. Subsequently a mixture of 128 oligonucleotides was aynthesized containing all possible encoding sequences of this heptapeptide, but excluding the third nucleotide of the last codon. The probe was radiolabelled with ³²P at its 5' end by means of T4 polynucleotide kinase and hybridized to a dot-blot containing sufficient cloned restriction fragments to cover the whole of the viral genome. Specific hybridization was obtained in the EcoRI B fragment. Determination of the nucleotide sequence of this fragment was done following the chain terminator method, as described by Sanger (PNAS, 74, 5463, (1977.)) Sequencing revealed the existence of a single open reading frame which enclodes a protein containing 646 amino acids with a molecular weight calculated according to electrophoretic mobility estimates of the protein (72 k.)

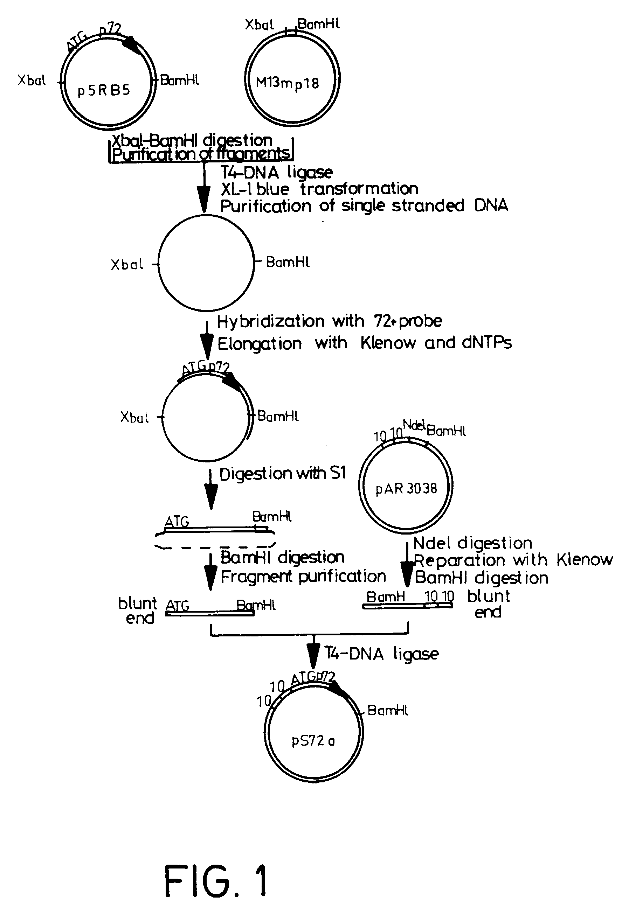

Once the protein p72 had been identified and sequenced, the next task was its expression in E. coli. The aim pursued here was to obtain maximum quantities of an antigen which would more closely resemble the viral protein. The system used was based on the E.coli strain BL21(DE3) and the plasmid pAR3038. BL21(DE3) is a lysogen of a derivative of bacteriophage lambda D69, which contains the gene of T7 RNA polymerase under the control of the IPTG-inducible promoter lacUV5 (Studier et al., J. Mol. Biol., 189, 113, (1986.)) The plasmid pAR3038 is a pBR322 derivative containing promoter ■Φ10 of the T7 bacteriorphage (Rosenberg et al., Gene, 56, 125, (1987.)) The protein p72 gene was inserted into the pAR3038 expression vector subsequent to promoter ■Φ10, following the strategy described below and diagrammatically represented in Fig. 1. An Xbal-Bam HI restriction fragment of approximately 3.5 kb, which contained the complete p72 gene and preceded by a sequence of 450 pb, was subcloned in the bacteriorphage vector M 13mp18 yielding a single-stranded DNA. Subsequently this single stranded DNA was hybridized with a synthetic oligonucleotide corresponding to the 17 first nucleotides of the p72 gene. This oligonucleotide was used as a primer in order to obtain, by means of the Klenow fragment of E. coli polymerase I DNA, a partially double stranded DNA. It was digested with nuclease S1 to eliminate the single stranded regions, the nuclease being removed by phenol-chloroform extraction followed by BamHI digestion. The resulting fragment of approximately 3 kb was purified by means of electrophoresis in a low melting point agarose gel and linked to pAR3038 which has been subsequently treated with NdeI, the Klenow fragment and BamHI. The ligation mixture was used to transform the DH5 E. coli strain. The colonies corresponding to bacteria which had been transformed with carrier plasmids of the complete p72 gene were separated by means of hybridization with the oligonucleotide which had been used as a primer and radiolabelled at its 5' end with ³²P. Plasmidic DNA was isolated from the selected clones and subsequently sequenced. Among the recombinant plasmids thus built up the so-called pS72a type was selected after making sure that the complete gene had been correctly inserted adjacent to the promoter region of the vector. This plasmid was then used to transform the E. coli BL21 (DE 3) strain, due deposit of same being made at the "Collection Nationale de Cultures de Microorganismes" of the Pasteur Institute in Paris. The register assigned to the specimen is I-860 dated May 26, 1989. The bacteria transformed with pAR3038 and the recombinant plasmid pS72a (as well as its derivatives obtained by means of deletions from the non-codifying 3' region) were grown at 37° C under gentle stirring in an LB medium with 200 µg/ml ampicillin until an optical density of approximately 1-0 was reached at 600 nm. Then 1 mM IPTG was added and 45 minutes later rifampicin to achieve a final concentration of 160 ug/ml, which was incubated for another 45 minutes. The bacteria were collected through centrifuging, then resuspended in PBS and disrupted on a French press. The suspension with the disrupted cells was centrifuged at 15.000 rpm for 15 minutes at 4° C in a Beckman JA-20 rotor. The soluble as well as the insoluble fraction (resuspended in PBS) were distributed in lots and freezezstored at -20° C.

Once p72 production by a bacterial strain had been accomplished, the next objective of the invention addressed purification of the protein p72 produced in the bacteria for the purpose of obtaining the best quality antigen possible. To this end the insoluble fraction of the extracts is dissolved in SDS and purified preferably by means of HPLC. The ASF detection method consists of obtaining serum samples from animals suspected to suffer the disease and subjecting the samples to an antigen-antibody interaction test, preferably an enzyme-immunp-assay, utilizing the protein p72 obtained according to the procedure described above.

### DESCRIPTION OF THE FIGURES

Fig. 1. Strategy followed when building up plasmid pS72a.

Fig. 2 Purification of protein p72 from recombinant E. coli BL21(DE3) extracts. The bacterial extract was resuspended in 1% SDS and chromatographed on an equilibrated molecular size exclusion HPLC column and eluted with 0.1 M ammonium acetate, ph 5, 1% SDS, at a flow rate of 0.4 ml/min. In the upper part of the figure the electrophoresis data of fractions 48 to 50 are shown.

Fig. 3 ELISA titration of the purified protein. The amounts of protein indicated were bound to a microtiter plate and enzyme-immuno-assayed vis à vis sera from ASF-virus infected pigs (ASF+) as compared to non-infected controls (ASF-.)

### ABBREVIATIONS

- ELISA :: Enzyme-Immuno-Assay
- HPLC :: High Performance Liquid Chromatography
- IPTG :: Isopropyl B-D-Thiogalactoside
- Kb :: Kilobase
- Kd :: Kilodaltons
- OPD :: O-Phenylene-Diamine
- PBS :: Phosphate Buffer Saline
- ASF :: African Swine Fever
- PT :: 0.1% Tween in PBS
- PTG :: 0.1% Tween, 1% Gelatine in PBS
- PTHs :: Phenyl-Thio-Hydantoins
- rpm :: revolutions per minute
- SDS :: Sodium Dodecyl Sulphate
- Tris :: Tris-hydroxy-methyl-aminomethane

The following examples describe the invention to the sole purpose of further illustration. By no means, however, should they be taken as exhaustive.

### EXAMPLES

### EXAMPLE 1.-

### Demonstration of bacteria-produced protein p72

Protein p72 production in bacteria following the method described was initially demonstrated by Western analysis, according to Burnette's method (Anal-Biochem., 112, 195, (1981.)) To this end, the extracts obtained from recombinant bacteria were separated by electrophoresis in a polyacrylamide/SDS gel, placed onto nitronuclease filter and hybridized with ASFV-infected pig sera, as well as with non-infected controls. Western analysis revealed the existence of band corresponding to a 72 Kd protein, specifically recognized in the positive serum, which did not appear in the extracts from bacteria transformed with non-inserted pAR3038.

### EXAMPLE 2.-

### Purification of protein p72 from recombinant E. coli BL 21 (DE 3) extracts

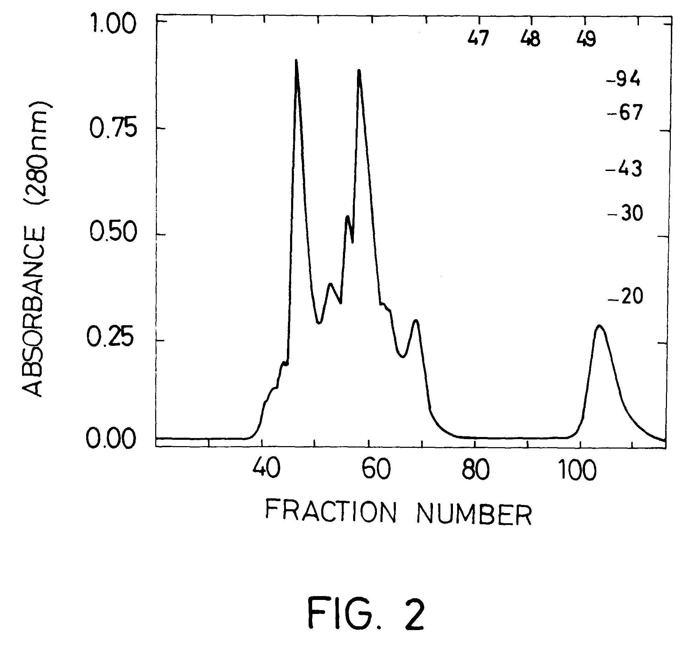

The insoluble fraction of the recombinant bacteria extracts was dissolved in 1% SDS and 0.1% mercaptoethanol, then boiled for 5 minutes and applied to a molecular size exclusion HPLC column. The chromatographic data are shown in Fig. 2. Electrophoretic analysis of the eluate revealed that fractions 48-50 contained the completely purified protein p72. The chromatographic behaviour of the recombinant protein coincides with that of the protein isolated from the infectious virus.

### EXAMPLE 3.-

### Assessment of the antigenic properties of recombinant protein p72

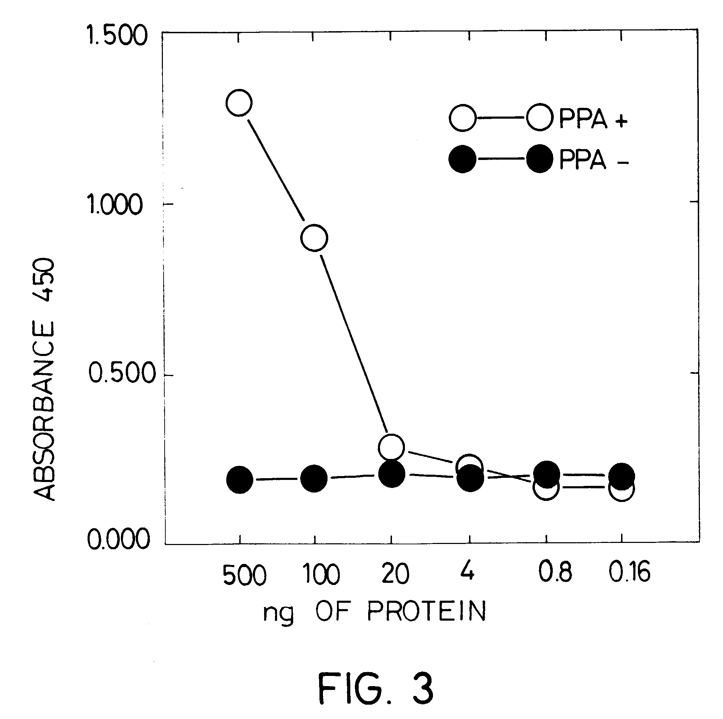

In order to assess the quality of the antigen obtained in the way described, different amounts of protein were fixed on amicrotiter plate and were brought to react witn the aforementioned antisera. Consequently, the antigens, diluted in PBS until reaching a final volume of 100 µl/well, were incubated overnight on microtiter plates (Costar, cat, 6595.) Incubation was done in hermetically sealed cuvettes which contained water-quenched filter paper in order to ensure a moist environment. After removing the sample residues, the plates were blocked for two hours with 200 µl PTG (0.1% Tween 20, 1% gelatine in PBS) per well, Subsequently the samples were incubated for one hour with the antibody diluted 50 times with PTG and washed 6 times with PT (0.1% Tween 20 in PBS.) Development was done with protein A conjugated with peroxidase. The product was washed again and then incubated for 5 minutes with 100 µl/ well 1 mg/ml OPD (O-phenylene diamine), and 1.2% H₂O₂ in a citrate-phosphate buffer, pH 5. The reaction was blocked with 3M H₂SO₄ and absorbance was measured at 450 nm. From the results obtained (Fig. 3) it may be concluded that under the experimental conditions 100 ng of recombinant protein p72 per well would be sufficient to ensure a fool-proof differentiation between infected and non-infected animal sera.

### EXAMPLE 4.-

### Immuno-enzyme assay of 17 pig sera with parallel use of E. coli produced protein p72 and a commercial antigen

Applying the procedure described in the preceding example reactivity of 17 pig sera was tested against the protein produced in our system and a commercial antigen marketed in ready for use form, i.e., fixed to the microtiter plates. The test conditions were identical for both antigens. Absorbance was measured at 450 nm, yielding the results listed below for each serum :

| | COMMERCIAL AGENT | | RECOMBINANT p72 | |
|---|---|---|---|---|
| SERUM | A | DIAGNOSIS | A | DIAGNOSIS |
| 1 | 0.797 | POSITIVE | 0.978 | POSITIVE |
| 2 | 0.791 | POSITIVE | 1.084 | POSITIVE |
| 3 | 0.775 | POSITIVE | 0.945 | POSITIVE |
| 4 | 0.162 | NEGATIVE | 0.190 | NEGATIVE |
| 5 | 0.186 | NEGATIVE | 0.223 | NEGATIVE |
| 6 | 0.560 | DOUBTFUL | 0.160 | NEGATIVE |
| 7 | 0.142 | NEGATIVE | 0.205 | NEGATIVE |
| 8 | 0.165 | NEGATIVE | 0.220 | NEGATIVE |
| 9 | 0.781 | POSITIVE | 0.928 | POSITIVE |
| 10 | 0.758 | POSITIVE | 1.057 | POSITIVE |
| 11 | 0.791 | POSITIVE | 0.928 | POSITIVE |
| 12 | 0.124 | NEGATIVE | 0.234 | NEGATIVE |
| 13 | 0.131 | NEGATIVE | 0.243 | NEGATIVE |
| 14 | 0.116 | NEGATIVE | 0.218 | NEGATIVE |
| 15 | 0.178 | NEGATIVE | 0.249 | NEGATIVE |
| CONTROL | +0.791 | POSITIVE | 1.079 | POSITIVE |
| CONTROL | -0.151 | NEGATIVE | 0.246 | NEGATIVE |

## Claims

1. A process for obtaining a test method for African Swine Fever Virus (ASFV) comprising the following stages
(a) purifying protein p72 from a viral capside and obtaining partial amino acid sequence T-P-D-D-P-G-A-M-M-I-T-F
(b) identifying the gene encoding said protein by means of radioactively labelled oligonucleotide probe containing an oligonucleotide mixture, whose sequence has been derived from the partial amino acids sequence obtained under (a) above;
(c) identifying the complete nuecleotide sequence of said gene;
(d) expressing said gene in E.coli BL21(DE3) by means of a pAR3038 plasmid whereby the p72 protein located in EcoRIB fragment of the genome of the African Swine Fever Virus is expressed by cloning thereof in said plasmid, to obtain a recombinant pS72a plasmid, and transforming said plasmid with said bacterial host;
(e) culturing said recombinant plasmid and purifying protein p72 from the extracts obtained;
(f) binding the purified protein onto microtiter plates and incubating it together with pig sera samples to be tested as to the presence of antibodies against African Swine Fever Virus.

2. A process according to claim 1, characterized in that the major protein of the capsid, p72, is purified by high performance liquid chromatography from the extracellular virus.

3. A process according to claim 1 or 2, characterized in that the partial amino acid sequence T-P-D-D-P-G-A-M-M-I-T-F of the protein p72 is obtained from a peptide resulting from enzyme digestion of the protein p72 purified by high performance liquid chromatography.

4. A process according to claims 1 to 3, characterized in that the identification of the protein p72 gene is achieved by means of the use of synthetic oligonucleotides whose sequence derives from the amino acid sequence obtained.

5. A process according to any of claims 1 to 4, characterized in that the recombinant bacteria obtained in the preceding stage are grown in a liquid medium which induced protein p72 production, the latter being purified from said bacterial extracts by means of high performance liquid chromatography.

6. A process according to any of claims 1 to 5, characterized in that the purified recombinant protein p72 obtained in the preceding stage is fixed onto microtiter plates (100ng/well), incubated for 20 hours at room temperature on hermetically sealed plates; after removing sample residues the plates are blocked for 2 hours with 200 µl PTG per well; they are incubated under the same conditions for 1 hour with the problem serum diluted 50 times with PTG, washed with PT and incubated with protein A conjugated with peroxidase; they are washed with PT and incubated for 5 minutes with 100 µl/well 1 mg/ml OPD and 1.2% H₂O₂ in citrate-phosphate buffer, pH 5; the reaction is blocked with 3M H₂SO₄, absorbance being measured at 450 nm.

7. A process according to claim 6, characterized in that the detection of anti-p72 antibodies is done by using a secondary antibody conjugated with peroxidase, alkaline phosphatase, β-galactosidase or another suitable enzyme.

## Patentansprüche

1. Verfahren zum Erhalt eines Testverfahrens auf Afrikanischen Schweinefiebervirus (ASFV), umfassend die folgenden Stufen
(a) Reinigen von Protein p72 aus einem viralen Capsid und Erhalten der Teilaminosäuresequenz T-P-D-D-P-G-A-M-M-I-T-F
(b) Identifizieren des dieses Protein kodierenden Gens mittels einer radioaktiv markierten Oligonukleotidsonde, enthaltend eine Oligonukleotidmischung, deren Sequenz von der unter (a) oben erhaltenen Teilaminosäuresequenz abgeleitet ist;
(c) Identifizieren der kompletten Nukleotidsequenz des Gens;
(d) Exprimieren des Gens in *E.coli* BL21(DE3) mittels eines pAR3038-Plasmids, wodurch das in EcoRIB-Fragment des Genoms des Afrikanischen Schweinefiebervirus lokalisierte p72-Protein durch Klonieren desselben in dem Plasmid unter Erhalt eines rekombinanten pS72a-Plasmids und Transformieren des Plasmids mit dem Bakterienwirt exprimiert wird;
(e) Kultivieren des rekombinanten Plasmids und Reinigen von Protein p72 aus den erhaltenen Extrakten;
(f) Binden des gereinigten Proteins auf Mikrotiterplatten und Inkubieren zusammen mit auf das Vorliegen von Antikörpern gegen Afrikanischen Schweinefiebervirus zu testenden Schweineserumproben.

2. Verfahren nach Anspruch 1, dadurch **gekennzeichnet,** daß das Hauptprotein des Capsids, p72, durch Hochdruckflüssigkeits-Chromatographie aus dem extrazellulären Virus gereinigt wird.

3. Verfahren nach Anspruch 1 oder 2, dadurch **gekennzeichnet,** daß die Teilaminosäuresequenz T-P-D-D-P-G-A-M-M-I-T-F des Proteins p72 aus einem Peptid erhalten wird, das aus dem Enzymverdau des durch Hochdruckflüssigchromatographie gereinigten p72-Proteins stammt.

4. Verfahren nach den Ansprüchen 1 bis 3, dadurch **gekennzeichnet,** daß die Identifikation des Proteins p72-Gens mittels der Verwendung von synthetischen Oligonukleotiden erfolgt, deren Sequenz aus der erhaltenen Aminosäuresequenz abgeleitet ist.

5. Verfahren nach Anspruch 1 bis 4, dadurch **gekennzeichnet,** daß die in der vorhergehenden Stufe erhaltenen rekombinanten Bakterien in einem flüssigen Medium, das die p72-Protein-Produktion induziert, kultiviert werden, wobei das letztere aus den bakteriellen Extrakten mittels Hochdruckflüssig-Chromatographie gereinigt wird.

6. Verfahren nach einem der Ansprüche 1 bis 5, dadurch **gekennzeichnet,** daß das gereinigte, in der vorhergehenden Stufe erhaltene rekombinante Protein p72 auf Mikrotiterplatten (100 ng/well) fixiert wird, 20 Stunden bei Raumtemperatur auf hermetisch versiegelten Platten inkubiert wird; nach Entfernen der Probereste werden die Platten 2 Stunden mit 200 µl PTG pro Well blockiert; sie werden dann unter den gleichen Bedingungen 1 Stunde mit dem 50-fach verdünnten Problem-Serum mit PTG inkubiert, mit PT gewaschen und mit dem Protein A , konjugiert an Peroxidase, inkubiert; sie werden mit PT gewaschen und 5 min mit 100 µl/well 1 mg/ml OPD und 1,2 % H₂O₂ in Citrat-Phosphatpuffer, pH 5, inkubiert; die Reaktion wird mit 3M H₂SO₄ blockiert und die Absorption bei 450 nm gemessen.

7. Verfahren nach Anspruch 6, dadurch **gekennzeichnet,** daß der Nachweis der Anti-p72-Antikörper unter Verwendung eines Sekundärantikörpers erfolgt, der mit Peroxidase, alkalischer Phosphatase, β-Galactosidase oder einem weiteren geeigneten Enzym konjugiert ist.

## Revendications

1. Un procédé d'obtention d'une méthode d'analyse pour détecter le virus de la peste porcine africaine (ASFV) comprenant les étapes suivantes :
(a) purifier la protéine p72 provenant d'une capside virale et obtenir la séquence partielle d'acides aminés T-P-D-D-P-G-A-M-M-I-T-F ;
(b) identifier le gène codant pour ladite protéine au moyen d'une sonde oligonucléotidique marquée radioactivement contenant un mélange d'oligonucléotides dont la séquence a été déduite de la séquence partielle d'acides aminés obtenue en (a) ci-dessus ;
(c) identifier la séquence nucléotidique complète dudit gène ;
(d) exprimer ledit gène dans *E. coli* BL21(DE3) au moyen d'un plasmide pAR3038 par lequel la protéine p72 située dans le fragment *Eco*RIB du génome du virus de la peste porcine africaine est exprimée par clonage de celui-ci dans ledit plasmide, pour obtenir un plasmide recombinant pS72a, et transformation dudit plasmide avec ledit hôte bactérien ;
(e) cultiver ledit plasmide recombinant et purifier la protéine p72 à partir des extraits obtenus ;
(f) fixer la protéine purifiée sur des plaques de microtitrage et la faire incuber avec des échantillons de sérum porcin à analyser pour déterminer la présence d'anticorps dirigé contre le virus de la peste porcine africaine.

2. Un procédé selon la revendication 1, caractérisé en ce que la protéine principale de la capside, p72, est purifiée par chromatographie en phase liquide à haute performance à partir du virus extracellulaire.

3. Un procédé selon la revendication 1 ou 2, caractérisé en ce que la séquence partielle d'acides aminés T-P-D-D-P-G-A-M-M-I-T-F de la protéine p72 est obtenue à partir d'un peptide résultant d'une digestion enzymatique de la protéine p72 purifiée par chromatographie en phase liquide à haute performance.

4. Un procédé selon les revendications 1 à 3, caractérisé en ce que l'identification du gène de la protéine p72 est réalisée en utilisant des oligonucléotides synthétiques dont la séquence est déduite de la séquence d'acides aminés obtenue.

5. Un procédé selon l'une quelconque des revendications 1 à 4, caractérisé en ce que les bactéries recombinantes obtenues dans l'étape précédente sont mises à croître dans un milieu liquide qui induit la production de protéine p72, cette dernière étant purifiée à partir des extraits bactériens par chromatographie en phase liquide à haute performance.

6. Un procédé selon l'une quelconque des revendications 1 à 5, caractérisé en ce que la protéine p72 recombinante purifiée obtenue dans l'étape précédente est fixée sur des plaques de microtitrage (100 ng/puits), incubée pendant 20 heures à la température ambiante sur des plaques fermées hermétiquement ; après enlèvement des résidus d'échantillon, les plaques sont soumises à un blocage pendant 2 heures avec 200 µl de PTG par puits ; elles sont incubées dans les mêmes conditions pendant 1 heure avec le sérum à analyser dilué 50 fois avec PTG, lavées avec PT et incubées avec de la protéine A conjuguée à la peroxydase ; elles sont lavées avec PT et incubées pendant 5 minutes avec 100 µl/puits de OPD à 1 mg/ml et 1,2 % de H₂O₂ dans du tampon citrate-phosphate, pH 5 ; la réaction est arrêtée avec H₂SO₄ 3M, l'absorbance étant mesurée à 450 nm.

7. Un procédé selon la revendication 6, caractérisé en ce que la détection d'anticorps anti-p72 est effectuée en utilisant un anticorps secondaire conjugué à la peroxydase, la phosphatase alcaline, la β-galactosidase ou une autre enzyme appropriée.
